# EUROPÄISCHE PATENTSCHRIFT

(11) **EP 1 323 829 B1**
(45) Veröffentlichungstag und Bekanntmachung des Hinweises auf die Patenterteilung: **31.05.2006**
(21) Anmeldenummer: 02026849.6
(22) Anmeldetag: 29.11.2002
(51) Int. Cl.: C12P 13/00, C12N 9/88

(54) **Verfahren zur Herstellung von geschützten, enantiomeren-angereicherten Cyanhydrinen durch in-situ-Derivatisierung**
Method for the preparation of protected, enantiomerically enriched cyanohydrines by in-situ derivatisation
Procédé de préparation de cyanohydrines pures et énantiomériquement enrichies par dérivatisation in-situ

(30) Priorität: 28.12.2001 AT 20442001
(43) Veröffentlichungstag der Anmeldung: 02.07.2003
(73) Patentinhaber: DSM Fine Chemicals Austria Nfg GmbH & Co KG, 4021 Linz (AT)
(72) Erfinder: Skranc, Wolfgang, 1220 Wien (AT); Pöchlauer, Peter, 4040 Linz (AT); Wirth, Irma, 4470 Enns (AT); Neuhofer, Rudolf, 4210 Mittertreffling (AT); Mayrhofer, Herbert, 4210 Engerwitzdorf (AT)
(74) Vertreter: Lindinger, Ingrid

(56) Entgegenhaltungen:
- OGNYANOV V I ET AL: "PREPARATION OF CHIRAL CYANOHYDRINS BY AN OXYNITRILASE-MEDIATED TRANSCYANATION" JOURNAL OF THE AMERICAN CHEMICAL SOCIETY, AMERICAN CHEMICAL SOCIETY, WASHINGTON, DC, US, Bd. 113, Nr. 18, 1991, Seiten 6992-6996, XP002165560 ISSN: 0002-7863
- GRIENGL H ET AL: "The synthesis of chiral cyanohydrins by oxynitrilases" TRENDS IN BIOTECHNOLOGY, ELSEVIER, AMSTERDAM, NL, Bd. 18, Nr. 6, Juni 2000 (2000-06), Seiten 252-256, XP004203650 ISSN: 0167-7799

## Beschreibung

Cyanhydrine sind etwa zur Synthese von alpha-Hydroxysäuren, alpha-Hydroxyketonen, beta-Aminoalkoholen, die zur Gewinnung biologisch wirksamer Stoffe, z. B. pharmazeutischer Wirkstoffe, Vitamine oder auch pyrethroider Verbindungen Verwendung finden, von Bedeutung.

Die Herstellung eines Cyanhydrins kann durch Anlagerung von Blausäure (HCN) an die Carbonylgruppe eines Aldehyds oder eines Ketons erfolgen, wobei Enantiomerengemische unsymmetrischer Cyanhydrine entstehen.
Viele Verfahren beruhen darauf, die Anlagerung von HCN an die Carbonylgruppe in Gegenwart eines chiralen Katalysators, beispielsweise einer Hydroxynitrillyase durchzuführen.
Da HCN jedoch eine äußerst giftige Substanz ist, wird ständig versucht den direkten Einsatz bzw. das direkte Handling zu vermeiden. Als Alternativen zu HCN wurden bisher beispielsweise Cyanhydrine der allgemeinen Formel RR'C(OH)(CN), wobei R und R' unabhängig voneinander Wasserstoff oder eine unsubstituierte Kohlenwasserstoffgruppe, oder gemeinsam eine Alkylengruppe mit 4 oder 5 C-Atomen, wobei R und R' nicht gleichzeitig Wasserstoff bedeuten, wie etwa Acetocyanhydrin, als Cyanidgruppendonor eingesetzt.
Einen weiteren Cyanidgruppendonor stellt beispielsweise Trimethylsilylcyanid dar, der gemäß J. Am. Chem. Soc. 2001, 123, 9908-9909 mit Zuckerderivaten bei -40°C in einem absoluten Alkohol umgesetzt wird.

Ein weiteres Problem in der Herstellung von Cyanhydrinen ist, dass Cyanhydrine an sich unbeständig sind und dazu neigen, sich in Umkehr ihrer Bildungsreaktion zu zersetzen, sodass bereits versucht wurde diese durch die unterschiedlichsten Zusätze, insbesondere von Säuren, wie etwa Schwefelsäure, Phosphorsäure, HCI, Toluolsulfonsäure, Essigsäure, Propionsäure u.s.w. zu stabilisieren.

Bei manchen Cyanhydrinen, wie etwa bei Acetophenonderivaten, ist außerdem die Gleichgewichtslage der Reaktion ziemlich ungünstig, wodurch diese Cyanhydrine nur in schlechten Ausbeuten erhalten werden.

Aufgabe der vorliegenden Erfindung war es, ein Hydroxynitrillyase-katalysiertes Verfahren zur Herstellung von stabilen, enantiomerenangereicherten Cyanhydrinen zu finden, bei welchem der direkte Einsatz von Blausäure vermieden wird und das eine Gleichgewichtsverschiebung zur Erzielung hoher Konversionen ermöglicht.

Unerwarteterweise konnte diese Aufgabe durch ein Verfahren gelöst werden, bei welchem Kohlensäureesternitrile als Cyanidgruppendonoren eingesetzt werden, wodurch eine in-situ-Derivatisierung und somit Stabilisierung der enantiomerenangereicherten Cyanhydrine erfolgt.

Gegenstand der vorliegenden Erfindung ist demnach ein Verfahren zur Herstellung von geschützten, enantiomeren-angereicherten Cyanhydrinen der Formel 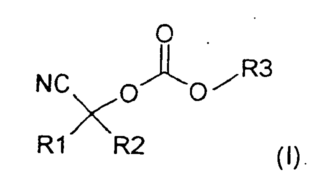 in der R1 und R2 unabhängig voneinander einen gegebenenfalls ein- oder mehrfach substituierten C₁-C₂₀-Alkyl-, C₅-C₂₀-Aryl-, C₅-C₂₀-Heteroaryl-, C₅-C₂₀-Alkaryl- C₅-C₂₀₋Alkylheteroaryl- oder C₅-C₂₀-Aralkylrest oder einen gegebenenfalls ein- oder mehrfach substituierten C₅-C₂₀-Heterocyclus oder C₅-C₂₀-Alkylheterocyclus oder gemeinsam einen gegebenenfalls substituierten C₄-C₂₀-Alkylenrest, der ein oder mehrere Heteroatome in der Kette enthalten kann bedeuten können, oder einer der Reste Wasserstoff bedeutet, und R3 ein gegebenenfalls substituierter C₁-C₂₀-Alkyl-, C₅-C₂₀₋Aryl- oder C₅-C₂₀-Heteroarylrest sein kann, das dadurch gekennzeichnet ist, dass ein Aldehyd oder Keton der Formel 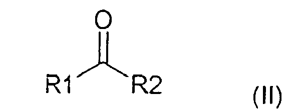 in der R1 und R2 wie oben definiert sind, in Gegenwart einer (R)- oder (S)-Hydroxynitrillyase im organischen, wässrigen oder 2-Phasensystem oder in Emulsion bei einer Temperatur von -5 bis +40°C mit einem Kohlensäureesternitril der Formel 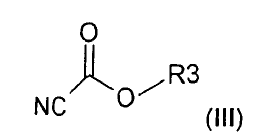 in der R3 wie oben definiert ist,
zu den entsprechenden O-geschützten, enantiomeren-angereicherten Cyanhydrinen der Formel (I) umgesetzt werden.

Bei dem erfindungsgemäßen Verfahren werden Aldehyde oder Ketone der Formel (II) als Edukte verwendet.
In der Formel (II) können R1 und R2 unabhängig voneinander einen gegebenenfalls ein- oder mehrfach substituierten C₁-C₂₀-Alkyl-, C₅-C₂₀-Aryl-, C₅-C₂₀-Heteroaryl-, C₅₋C₂₀-Alkaryl-, C₅-C₂₀- Alkylheteroaryl- oder C₅-C₂₀-Aralkylrest oder einen gegebenenfalls ein- oder mehrfach substituierten C₅-C₂₀-Heterocyclus oder C₅-C₂₀₋Alkylheterocyclus bedeuten.

Unter C₁-C₂₀-Alkyl sind dabei gesättigte oder ein- oder mehrfach ungesättigte, lineare, verzweigte oder cyclische, primäre, sekundäre oder tertiäre Hydrocarbonreste zu verstehen. Dies sind beispielsweise C₁-C₂₀-Alkylreste, wie etwa Methyl, Ethyl, Propyl, iso-Propyl, Butyl, iso-Butyl, t-Butyl, Butenyl, Butinyl, Pentyl, Cyclopentyl, iso-Pentyl, neo-Pentyl, Pentenyl, Pentinyl, Hexyl, iso-Hexyl, Cyclohexyl, Cyclohexylmethyl, 3-Methylpentyl, 2,2-Dimethylbutyl, 2,3-Dimethylbutyl, Octyl, Cyclooctyl, Decyl, Cyclodecyl, Dodecyl, Cyclododecyl u.s.w. Bevorzugt sind dabei C₁-C₁₂-Alkylreste und besonders bevorzugt C₁-C₈-Alkylreste. Die Alkylgruppe kann gegebenenfalls ein- oder mehrfach durch unter den Reaktionsbedingungen inerte Gruppen substituiert sein. Geeignete Substituenten sind beispielsweise gegebenenfalls substituierte Aryl- oder Heteroarylgruppen, wie Phenyl-, Phenoxy- oder Indolylgruppen, Halogen-, Hydroxy-, Hydroxy-C₁-C₅-Alkyl, C₁-C₆₋Alkoxy-, Aryloxy-, bevorzugt C₆-C₂₀-Aryloxy-, C₁-C₆-Alkylthio-,- Amino-, Alkylamino-, bevorzugt C₁-C₆-Alkylamino-, Arylamino-, bevorzugt C₆-C₂₀-Arylamino-, Ether-, Thioether, Carbonsäureester-, Carbonsäureamid-, Sulfoxid-, Sulfon-, Sulfonsäure, Sulfonsäureester-, Sulfinsäure-,Mercaptan-, Nitro- oder Azidogruppen.

Unter Aryl sind bevorzugt C₆-C₂₀-Arylgruppen zu verstehen, wie etwa Phenyl, Biphenyl, Naphthyl, Indenyl, Fluorenyl u.s.w.

Die Arylgruppe kann dabei gegebenenfalls ein- oder mehrfach substituiert sein. Geeignete Substituenten sind dabei wiederum gegebenenfalls substituierte Aryl- oder Heteroarylgruppen, wie Phenyl-, Phenoxy- oder Indolylgruppen, Halogen-, Hydroxy-, Hydroxy-C₁-C₅-Alkyl, C₁-C₆-Alkoxy-, Aryloxy-, bevorzugt C₆-C₂₀-Aryloxy-, C₁-C₆₋Alkylthio-, Amino-, Alkylamino-, bevorzugt C₁-C₆-Alkylamino-, Arylamino-, bevorzugt C₆-C₂₀-Arylamino-, Ether-, Thioether, Carbonsäureester-, Carbonsäureamid-, Sulfoxid-, Sulfon-, Sulfonsäure, Sulfonsäureester-, Sulfinsäure-,Mercaptan-, Nitro- oder Azidogruppen.

Unter Alkaryl oder Alkylaryl sind Alkylgruppen zu verstehen, die einen Arylsubstituenten aufweisen.
Aralkyl oder Arylalkyl bezieht sich auf eine Arylgruppe mit einem Alkylsubstituenten.

Unter Heteroaryl oder Heterocyclus sind cyclische Reste zu verstehen die zumindestens ein S-, O- oder N-Atom im Ring enthalten. Dies sind beispielsweise Furyl, Pyridyl, Pyrimidyl, Thienyl, Isothiazolyl, Imidazolyl, Tetrazolyl, Pyrazinyl, Benzofuranyl, Benzothiophenyl, Chinolyl, Isochinolyl, Benzothienyl, Isobenzofuryl, Pyrazolyl, Indolyl, Isoindolyl, Benzoimidazolyl, Purinyl, Carbazolyl, Oxazolyl, Thiazolyl, Isothiazolyl, 1,2,4-Thiadiazolyl, Isoxazolyl, Pyrrolyl, Chinazolinyl, Pyridazinyl, Phthalazinyl, Morpholinyl, u.s.w.
Funktionelle O- oder N-Gruppen können dabei nötigenfalls geschützt werden.
Die Heteroarylgruppe bzw. der Heterocyclus kann dabei gegebenenfalls ein- oder mehrfach durch die bereits oben angeführten Substituenten substituiert sein.

Unter Alkylheteroaryl bzw. Alkylheterocyclus sind dabei Alkylgruppen zu verstehen, die durch eine Heteroarylgruppe bzw. durch einen Heterocyclus substituiert sind.

Bevorzugt bedeuten R1 und R2 einen gesättigten, linearen oder verzweigten C₁-C₈₋Alkylrest, einen Benzyl- oder einen Phenylrest, wobei die Reste gegebenenfalls ein- oder mehrfach durch F, Cl, OH, Carbonsäurederivate, wie Carbonsäureester oder Carbonsäureamide, Amino, C₁-C₆-Alkylamino, C₆-C₂₀-Arylamino, C₁-C₆-Alkoxy, C₆₋C₂₀-Aryloxy, oder Nitro substituiert sein können.

R1 und R2 können aber auch gemeinsam einen gegebenenfalls substituierten C₄₋C₂₀-Alkylenrest, der ein oder mehrere Heteroatome aus der Gruppe O, N oder S oder eine NR4R5-Gruppe, wobei R4 und R5 unabhängig voneinander H oder C₁-C₆₋Alkylsein können, in der Kette enthalten kann bedeuten. In diesem Fall stellen die Edukte cyclische Ketone dar.
Bevorzugt sind C₄-C₇-Alkylenreste, die in Abhängigkeit von der Ringgröße des cyclischen Ketons höchstens 2 Heteroatome in der Alkylkette aufweisen. Der Alkylenrest kann weiters noch, wiederum in Abhängigkeit von der Ringgröße des cyclischen Ketons noch ein oder 2 Doppelbindungen aufweisen, wobei bei einem 5er-Ring diese nicht in Konjugation zur Carbonylgruppe stehen darf.
Der Alkylenrest kann zudem ein oder mehrfach durch die oben angeführten Reste substituiert sein.

Bei den eingesetzten Edukten kann jedoch auch einer der Reste R1 und R2 Wasserstoff bedeuten. In diesem Fall handelt es sich bei den Edukten um Aldehyde.

Erfindungsgemäß wird das gewünschte Edukt mit einem Kohlensäureesternitril der Formel (III) umgesetzt.
In der Formel (III) bedeutet R3 einen gegebenenfalls substituierten C₁-C₂₀-Alkyl-, C₅₋C₂₀-Aryl- oder C₅-C₂₀-Heteroarylrest. Der Alkylrest kann dabei gesättigt, ein- oder mehrfach ungesättigt, linear, verzweigt oder cyclisch sein. Die Arylreste und Heteroarylreste sind dabei wie oben definiert. Bevorzugt bedeutet R3 einen C₁-C₁₂-Alkylrest.
Geeignete Substituenten sind beispielsweise Phenyl, C₁-C₆-Alkyl, OH, Halogen oder eine Sulfoxygruppe.
Beispiele für geeignete Nitrile der Formel (III) sind Cyanameisensäuremethylester, -ethylester, -2,2,2-trichlorethylester, -tert. butylester, -benzylester, -allylester, -i-butylester, -2-ethylhexylester, -p-menthylester, u.s.w.

Kohlensäureesternitrile der Formel (III) sind käuflich erwerbbar oder können beispielsweise aus den entsprechenden Halogeniden und HCN oder einem Alkalicyanid, wie etwa in EP 0 136 145, Tetrahedron Letters No. 27, S.2517 oder J.Chem. Soc. Perkin Trans. 1, (15), 1729-35, beschrieben, 1993 hergestellt werden.

Pro Mol eingesetzte Aldehyd- oder Ketogruppe werden mindestens 1 Mol, bevorzugt 1 bis 5 Mole, besonders bevorzugt 2 bis 4 Mole, Kohlensäurenitril zugegeben.

Die erfindungsgemäße Reaktion findet im organischen, wässrigen oder 2-Phasensystem oder in Emulsion in Anwesenheit einer Hydroxynitrillyase als Katalysator statt.
Dabei wird bei der enantioselektiven Umsetzung im wässrigen System eine wässrige, die entsprechende HNL enthaltende Lösung oder Pufferlösung verwendet. Beispiele dafür sind Acetatpuffer, Boratpuffer, Phthalatpuffer, Citratpuffer, Phosphatpuffer u.s.w. oder Gemischen dieser Pufferlösungen.
Der pH-Wert dieser Lösung liegt dabei bei pH 2 bis 8, bevorzugt bei pH 2,5 bis 6,5, liegen.

Als organisches Verdünnungsmittel können mit Wasser nicht oder geringfügig mischbare aliphatische oder aromatische Kohlenwasserstoffe, die gegebenenfalls halogeniert sind, Alkohole, Ether oder Ester oder Gemische davon verwendet werden. Bevorzugt werden Methyl -tert. Butylether (MTBE), Diisopropylether, Dibutylether und Ethylacetat oder deren Gemische eingesetzt.
Die Umsetzung kann jedoch auch in einem Zweiphasensystem oder in Emulsion mit erfolgen.

Als HNLs eignen sich sowohl native als auch rekombinante (R)- und (S)-HNLs, die entweder als solche oder immobilisiert vorliegen.

Als (S)-Hydroxynitrillyase (HNL) kommen native (S)-Hydroxynitrillyasen z.B. aus Maniok und Hevea brasiliensis, sowie rekombinante (S)-HNL in Frage. Bevorzugt wird als native HNL HNL aus Hevea brasiliensis verwendet. Geeignete rekombinante (S)-HNL wird beispielsweise aus gentechnisch modifizierten Mikroorganismen, wie etwa Pichia pastoris, E. coli oder Saccharomyces cerevisiae erhalten.
Bevorzugt wird rekombinante (S)-Hnl aus Pichia pastoris eingesetzt.

Als (R)-HNL kommen beispielsweise (R)-Hydroxynitrillyasen aus Prunus amygdalus, Prunus laurocerasus oder Prunus serotina, oder rekombinante (R)-Hnl in Frage. Bevorzugt wird (R)-Hydroxynitrilase aus Prunus amygdalus oder eine rekombinante (R)-HNL verwendet.

Geeignete (R)- und (S)-HNLs sind beispielsweise aus WO 97/03204; EP 0 969 095; EP 0 951 561, EP 0 927 766, EP 0 632 130, EP 0547 655, EP 0 326 063, WO 01/44487 usw. bekannt.

Pro g Aldehyd bzw. Keton werden etwa 10 bis 20 000 IU Aktivität, bevorzugt etwa 100 bis 10 000 IU Aktivität, Hydroxynitrillyase zugesetzt.

Die Reaktionstemperaturen liegt bei etwa -5 bis +40°C, bevorzugt bei etwa 0 bis 30°C.

Bevorzugt wird die erfindungsgemäße Reaktion im wässrigen System durchgeführt, wobei zuerst die entsprechende HNL als wässrige Lösung vorgelegt, in Abhängigkeit von der gewählten HNL mittels einer geeigneten Säure, beispielsweise mittels Citronensäure oder mit einem Puffer, wie etwa Acetatpuffer, Boratpuffer, Phthalatpuffer, Citratpuffer, Phosphatpuffer u.s.w. oder Gemischen dieser Pufferlösungen, auf den gewünschten pH gestellt wird. Anschließend wird das entsprechende Edukt der Formel (II) zugesetzt und die Reaktion durch Zugabe des Kohlensäureesternitrils der Formel (III) gestartet. Dabei entwickelt sich HCN das unter HNL-katalysierter Addition mit dem eingesetzten Edukt zuerst zu einem korrespondierenden enantiomerenangereicherten Cyanhydrin reagiert. Restliches Kohlensäurenitril reagiert mit dem enantiomerenangereicherten Cyanhydrin zu dem stabilen, O-geschützten, enantiomerenangereicherten Cyanhydrin der Formel (I), wobei wieder HCN frei wird, die wiederum zur Cyanhydrinbildung verwendet wird.
Es kann jedoch auch zuerst das Edukt vorgelegt werden und anschließend die entsprechende HNL als wässrige Lösung zugegeben werden.

Der Reaktionsverlauf ist aus folgendem Schema ersichtlich:

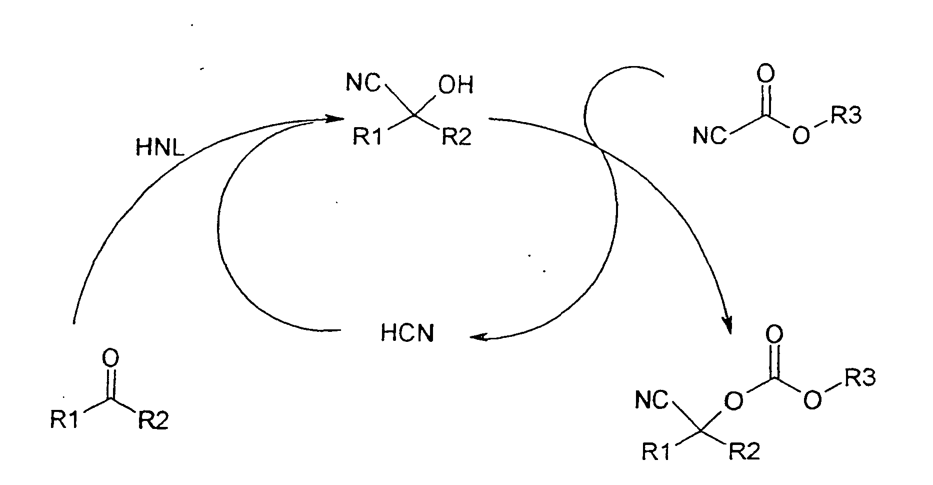

Das erfindungsgemäße Verfahren ermöglicht dabei durch die chemische O-Derivatisierung eine Gleichgewichtsverschiebung auf die Seite des gewünschten Endproduktes, wodurch insbesondere bei Cyanhydrinen mit ursprünglich ungünstiger Gleichgewichtslage, wie etwa Acetophenonderivate, im Vergleich zum Stand der Technik eine wesentlich höhere Konversion erzielt werden kann. Gleichzeitig wird durch die Derivatisierung eine Stabilisierung der gebildeten Cyanhydrine erreicht. Ein weiterer Vorteil des erfindungsgemäßen Verfahrens ist die in situ Generierung von HCN und die ständige Nachlieferung von HCN, wobei gleichzeitig ein direktes Einsetzen von HCN vermieden wird. Das Derivatisierungsreagens setzt dabei unerwarteterweise die Aktivität der eingesetzten HNL nicht oder nur unbedeutend herab.

### Beispiel 1:

### HNL-katalysierte Reaktion von Cyanameisensäureethylester mit Benzaldehyd:

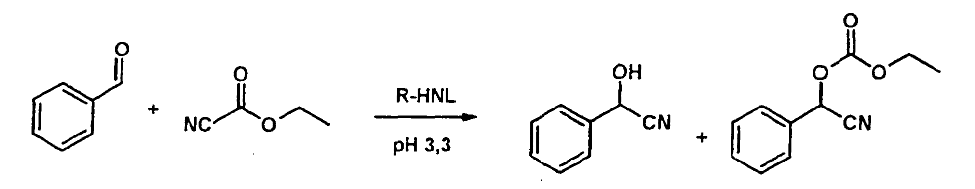

Es wurden 2,5 ml rekombinante R-HNL Lösung (300 iU/ml) mit einer Citronensäurelösung auf pH 3,3 eingestellt und mit 2,5 ml 50 mmol Kaliumphosphat / Citratpuffer pH 3,3 verdünnt. Anschließend wurden 106 mg (1 mmol) Benzaldehyd zugesetzt und die Reaktion durch Zugabe von 297 µl (3 mmol) Cyanameisensäureethylester gestartet. Das Reaktionsgemisch wurde bei 25 °C gerührt und die Bildung von Benzaldehydcyanhydrin und O-Ethoxycarbonyl-cyanhydrin mittels Gaschromatographie an einer chiralen Phase (Cyclodextrinsäule) verfolgt und die entsprechenden Enantiomerenreinheiten errechnet.

### Reaktionsverlauf:

| Reaktionszeit (Stunden) | Benzaldehyd | Benzaldehydcyanhydrin | | O-Ethoxycarbonyl-cyanhydrin | |
|---|---|---|---|---|---|
| | (Area%) | (Area%) | (%ee) | | |
| | | | | (Area%) | (%ee) |
| 1 | 61 | 38 | 99,9 | 1 | 99,9 |
| 3 | 15 | 77 | 99,5 | 8 | 99,9 |
| 23 | 2 | 65 | 93,7 | 33 | 99,9 |
| 44,5 * | < 1 | 34 | 89,4 | 66 | 94,7 |

| | | | | | |
|---|---|---|---|---|---|
| *Nach 26 Stunden wurden nochmals 297 µl (3 mmol) Cyanameisensäureethylester zugesetzt. | | | | | |

Identifizierung des O-Ethoxycarbonyl-cyanhydrines:
¹H-NMR in CDCl₃, 300 MHz: δ 1,30-1,32 (t, 3H), 4,21-4,36 (m, 2H), 6,27 (s, 1H) 7,39-7,57 (m, 5H)

## Patentansprüche

1. Verfahren zur Herstellung von geschützten, enantiomeren-angereicherten Cyanhydrinen der Formel 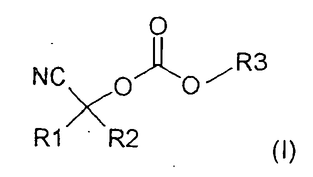 in der R1 und R2 unabhängig voneinander einen gegebenenfalls ein- oder mehrfach substituierten C₁-C₂₀-Alkyl-, C₅-C₂₀-Aryl-, C₅-C₂₀-Heteroaryl-, C₅-C₂₀-Alkaryl-, C₅-C₂₀- Alkylheteroaryl- oder C₅-C₂₀-Aralkylrest oder einen gegebenenfalls ein- oder mehrfach substituierten C₅-C₂₀-Heterocyclus oder C₅-C₂₀-Alkylheterocyclus oder gemeinsam einen gegebenenfalls substituierten C₄-C₂₀-Alkylenrest, der ein oder mehrere Heteroatome in der Kette enthalten kann bedeuten können, oder einer der Reste Wasserstoff bedeutet, und R3 ein gegebenenfalls substituierter C₁₋C₂₀-Alkyl-, C₅-C₂₀-Aryl- oder C₅-C₂₀-Heteroarylrest sein kann, das **dadurch gekennzeichnet ist, dass** ein Aldehyd oder Keton der Formel 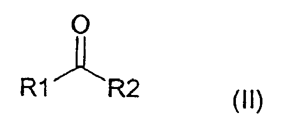 in der R1 und R2 wie oben definiert sind, in Gegenwart einer (R)- oder (S)-Hydroxynitrillyase im organischen, wässrigen oder 2-Phasensystem oder in Emulsion bei einer Temperatur von -5 bis +40°C mit einem Kohlensäureesternitril der Formel 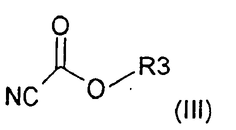 in der R3 wie oben definiert ist,
zu den entsprechenden O-geschützten, enantiomeren-angereicherten Cyanhydrinen der Formel (I) umgesetzt werden.

2. Verfahren nach Anspruch 1, **dadurch gekennzeichnet, dass** als Edukte Verbindungen der Formel (II) eingesetzt werden in der R1 und R2 unabhängig voneinander einen C₁-C₂₀-Alkyl-, C₅-C₂₀-Aryl-, C₅-C₂₀-Heteroaryl-, C₅-C₂₀-Alkaryl-, C₅-C₂₀₋Alkylheteroaryl- oder C₅-C₂₀-Aralkylrest oder einen gegebenenfalls ein- oder mehrfach substituierten C₅-C₂₀-Heterocyclus oder C₅-C₂₀-Alkylheterocyclus oder gemeinsam einen gegebenenfalls substituierten C₄-C₂₀-Alkylenrest, der ein oder mehrere Heteroatome in der Kette enthalten kann bedeuten können, wobei die Reste ein- oder mehrfach durch gegebenenfalls substituierte Aryl- oder Heteroarylgruppen, Halogen-, Hydroxy, Hydroxy-C₁-C₅-Alkyl, C₁-C₆-Alkoxy-, C₆-C₂₀₋Aryloxy-, C₁-C₆-Alkylthio-, Amino-, C₁-C₆-Alkylamino-, C₆-C₂₀-Arylamino-, Ether-, Thioether, Carbonsäureester-, Carbonsäureamid-, Sulfoxid-, Sulfon-, Sulfonsäure, Sulfonsäureester-, Sulfinsäure-, Mercaptan-, Nitro- oder Azidogruppen substituiert sein können,
oder einer der Reste Wasserstoff bedeutet.

3. Verfahren nach Anspruch 2, **dadurch gekennzeichnet, dass** als Edukte Verbindungen der Formel (II) eingesetzt werden in der R1 und R2 unabhängig voneinander einen gesättigten, linearen oder verzweigten C₁-C₈-Alkylrest, einen Benzyl- oder einen Phenylrest bedeuten, wobei die Reste gegebenenfalls ein- oder mehrfach durch F, Cl, OH, Carbonsäureester, Carbonsäureamide, Amino, C₁-C₆-Alkylamino, C₆-C₂₀-Arylamino, C₁-C₆-Alkoxy, C₆-C₂₀-Aryloxy, oder Nitro substituiert sein können oder einer der Reste Wasserstoff bedeutet.

4. Verfahren nach Anspruch 1, **dadurch gekennzeichnet, dass** als Kohlensäurenitril eine Verbindung der Formel (III) eingesetzt wird in der R3 einen C₁-C₂₀-Alkylrest, der durch einen oder mehreren Substituenten aus der Gruppe Phenyl, C₁-C₆₋Alkyl, OH, Halogen oder Sulfoxy substituiert sein kann.

5. Verfahren nach Anspruch 1, **dadurch gekennzeichnet, dass** bei der enantioselektiven Umsetzung im wässrigen System eine wässrige, die entsprechende Hydroxynitrillyase enthaltende Lösung oder eine Acetatpuffer, Boratpuffer, Phthalatpuffer-, Citratpuffer-, Phosphatpufferlösung oder ein Gemisch dieser Pufferlösungen verwendet wird.

6. Verfahren nach Anspruch 5, **dadurch gekennzeichnet, dass** in der wässrigen Lösung ein pH Wert von 2 bis 8 eingestellt wird.

7. Verfahren nach Anspruch 1, **dadurch gekennzeichnet, dass** als organisches Verdünnungsmittel mit Wasser nicht oder geringfügig mischbare aliphatische oder aromatische Kohlenwasserstoffe, die gegebenenfalls halogeniert sind, Alkohole, Ether oder Ester oder Gemische eingesetzt werden.

8. Verfahren nach Anspruch 1, **dadurch gekennzeichnet, dass** die Umsetzung jedoch auch in einem Zweiphasensystem oder in Emulsion erfolgt.

9. Verfahren nach Anspruch 1, **dadurch gekennzeichnet, dass** als Hydroxynitrillyase native oder rekombinante (R)- und (S)-Hydroxynitrillyasen eingesetzt werden, die entweder als solche oder immobilisiert vorliegen.

10. Verfahren nach Anspruch 9, **dadurch gekennzeichnet, dass** als Hydroxynitrillyase native (S)-Hydroxynitrillyasen aus Maniok und Hevea brasiliensis, rekombinante (S)-Hydroxynitrillyase aus gentechnisch modifizierten Mikroorganismen aus der Gruppe Pichia pastoris, E. coli oder Saccharomyces cerevisiae, native (R)-Hydroxynitrillyasen aus Prunus amygdalus, Prunus laurocerasus oder Prunus serotina oder rekombinante (R)-Hydroxynitrillyasen eingesetzt werden.

## Claims

1. Process for preparing protected, enantiomer-enriched cyanohydrins of the formula 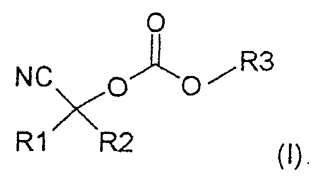 where R1 and R2 independently of one another can be an unsubstituted, monosubstituted or polysubstituted C₁-C₂₀-alkyl, C₅-C₂₀-aryl, C₅-C₂₀₋heteroaryl, C₅-C₂₀-alkaryl, C₅-C₂₀-alkylheteroaryl or C₅-C₂₀-aralkyl radical or an unsubstituted, monosubstituted or polysubstituted C₅-C₂₀₋heterocycle, or C₅-C₂₀-alkylheterocycle or together can be an unsubstituted or substituted C₄-C₂₀-alkylene radical, which can contain one or more heteroatoms in the chain, or one of the radicals is hydrogen, and R3 can be an unsubstituted or substituted C₁-C₂₀-alkyl, C₅-C₂₀₋aryl or C₅-C₂₀-heteroaryl radical, which process is **characterized in that** an aldehyde or ketone of the formula 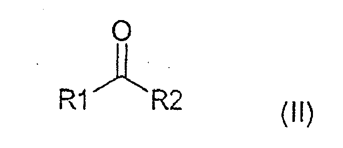 where R1 and R2 are defined as above, is reacted in the presence of an (R)- or (S)-hydroxynitrile lyase in an organic, aqueous or 2-phase system or in emulsion at a temperature of -5 to +40°C with a carbonic ester nitrile of the formula 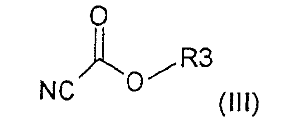 where R3 is defined as above,
to give the corresponding O-protected, enantiomer-enriched cyanohydrins of the formula (I).

2. Process according to Claim 1, **characterized in that** the starting materials used are compounds of the formula (II) where R1 and R2 independently of one another can be a C₁-C₂₀-alkyl, C₅-C₂₀-aryl, C₅-C₂₀-heteroaryl, C₅-C₂₀-alkaryl, C₅-C₂₀-alkylheteroaryl or C₅-C₂₀-aralkyl radical, or an unsubstituted, monosubstituted or polysubstituted C₅-C₂₀-heterocycle or C₅-C₂₀-alkylheterocycle or together can be an unsubstituted or substituted C₄-C₂₀-alkylene radical, which can contain one or more heteroatoms in the chain, where the radicals can be monosubstituted or polysubstituted by unsubstituted or substituted aryl or heteroaryl groups, halogen, hydroxyl, hydroxy-C₁-C₅-alkyl, C₁-C₆-alkoxy, C₆-C₂₀-aryloxy, C₁-C₆-alkylthio, amino, C₁-C₆-alkylamino, C₆-C₂₀-arylamino, ether, thioether, carboxylic ester, carboxamide, sulphoxide, sulphone, sulphonic acid, sulphonic ester, sulphinic acid, mercaptan, nitro or azido groups, or one of the radicals is hydrogen.

3. Process according to Claim 2, **characterized in that** the starting materials used are compounds of the formula (II) where R1 and R2 independently of one another are a saturated, unbranched or branched C₁-C₈-alkyl radical, a benzyl radical or a phenyl radical, where the radicals can be unsubstituted, monosubstituted or polysubstituted by F, Cl, OH, carboxylic esters, carboxamides, amino, C₁-C₆-alkylamino, C₆-C₂₀-arylamino, C₁-C₆₋alkoxy, C₆-C₂₀-aryloxy, or nitro, or one of the radicals is hydrogen.

4. Process according to Claim 1, **characterized in that** the carbononitrile used is a compound of the formula (III) where R3 is a C₁-C₂₀-alkyl radical which can be substituted by one or more substituents selected from the group consisting of phenyl, C₁-C₆-alkyl, OH, halogen or sulphoxy.

5. Process according to Claim 1, **characterized in that**, in the case of the enantioselective reaction in an aqueous system, an aqueous solution containing the corresponding hydroxynitrile lyase or an acetate buffer, borate buffer, phthalate buffer, citrate buffer, phosphate buffer solution or a mixture of these buffer solutions is used.

6. Process according to Claim 5, **characterized in that** a pH of 2 to 8 is established in the aqueous solution.

7. Process according to Claim 1, **characterized in that**, as organic diluent, water-immiscible or only slightly water-miscible aliphatic or aromatic hydrocarbons which may be halogenated, alcohols, ethers or esters or mixtures are used.

8. Process according to Claim 1, **characterized in that** the reaction, however, alternatively proceeds in a two-phase system or in emulsion.

9. Process according to Claim 1, **characterized in that** the hydroxynitrile lyases used are native or recombinant (R)- and (S)-hydroxynitrile lyases which are present either as such or immobilized.

10. Process according to Claim 9, **characterized in that** the hydroxynitrile lyases used are native (S)-hydroxynitrile lyases from manioc and Hevea brasiliensis, recombinant (S)-hydroxynitrile lyase from genetically modified microorganisms from the group Pichia pastoris, E. coli or Saccharomyces cerevisiae, native (R)-hydroxynitrile lyases from Prunus amygdalus, Prunus laurocerasus or Prunus serotina, or recombinant (R)-hydroxynitrile lyases.

## Revendications

1. Procédé de préparation de cyanhydrines protégées, énantiomériquement enrichies, de formule 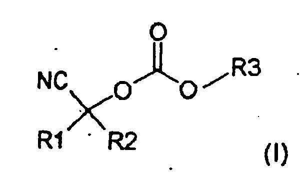 dans laquelle R1 et R2 peuvent représenter indépendamment l'un de l'autre un radical alkyle en C₁ à C₂₀, aryle en C₅ à C₂₀, hétéroaryle en C₅ à C₂₀, alkaryle en C₅ à C₂₀, alkylhétéroaryle en C₅ à C₂₀ ou aralkyle en C₅ à C₂₀ éventuellement substitué une ou plusieurs fois, ou un hétérocycle en C₅ à C₂₀ ou un alkylhétérocycle en C₅ à C₂₀ éventuellement substitué une ou plusieurs fois, ou représenter ensemble un radical alkylène en C₄ à C₂₀ éventuellement substitué qui peut contenir dans la chaîne un ou plusieurs hétéroatomes, ou bien l'un des radicaux représente de l'hydrogène, et R3 peut représenter un radical alkyle en C₁ à C₂₀, aryle en C₅ à C₂₀ ou hétéroaryle en C₅ à C₂₀ éventuellement substitué, **caractérisé en ce que** l'on fait réagir un aldéhyde ou une cétone de formule 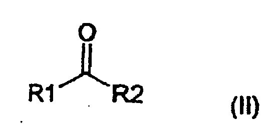 dans laquelle R1 et R2 sont tels que définis plus haut, en présence d'une (R)- ou (S)-hydroxynitrile lyase dans un milieu organique, aqueux ou biphasique ou en émulsion, à une température de -5 à +40°C, avec un nitrile ester d'acide carbonique de formule 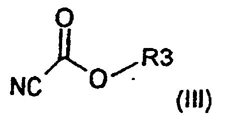 dans laquelle R3 est tel que défini plus haut,
pour former les cyanhydrines O-protégées correspondantes énantiomériquement enrichies de la formule (I).

2. Procédé selon la revendication 1, **caractérisé en ce que** l'on met en oeuvre en tant qu'éduits des composés de la formule (II) dans laquelle R1 et R2 représentent indépendamment l'un de l'autre un radical alkyle en C₁ à C₂₀, aryle en C₅ à C₂₀, hétéroaryle en C₅ à C₂₀, alkaryle en C₅ à C₂₀, alkylhétéroaryle en C₅ à C₂₀ ou aralkyle en C₅ à C₂₀, ou un hétérocycle en C₅ à C₂₀ ou un alkylhétérocycle en C₅ à C₂₀ éventuellement substitué une ou plusieurs fois, ou représentent ensemble un radical alkylène en C₄ à C₂₀ éventuellement substitué, qui peut contenir dans la chaîne un ou plusieurs hétéroatomes, les radicaux pouvant être substitués une ou plusieurs fois par des groupes aryle ou hétéroaryle éventuellement substitués, des groupes halogéno, hydroxy, hydroxy (alkyle en C₁-C₅), alcoxy en C₁ à C₆, aryloxy en C₆ à C₂₀, alkylthio en C₁ à C₆, amino, alkylamino en C₁-C₆, arylamino en C₆-C₂₀, éther, thioéther, ester d'acide carboxylique, amide d'acide carboxylique, sulfoxyde, sulfone, acide sulfonique, ester d'acide sulfonique, acide sulfinique, mercaptan, nitro ou azido, ou bien l'un des radicaux représente de l'hydrogène.

3. Procédé selon la revendication 2, **caractérisé en ce que** l'on met en oeuvre en tant qu'éduits des composés de la formule (II) dans laquelle R1 et R2 représentent indépendamment l'un de l'autre un radical alkyle en C₁ à C₈ saturé, linéaire ou ramifié, un radical benzyle ou phényle, les radicaux pouvant le cas échéant être substitués une ou plusieurs fois par F, Cl, OH, des groupes ester d'acide carboxylique, amide d'acide carboxylique, amino, alkylamino en C₁-C₆, arylamino en C₆-C₂₀, alcoxy en C₁ à C₆, aryloxy en C₆ à C₂₀ ou nitro, ou bien l'un des radicaux représente de l'hydrogène.

4. Procédé selon la revendication 1, **caractérisé en ce que** l'on met en oeuvre en tant que nitrile d'acide carbonique un composé de la formule (III) dans laquelle R3 représente un radical alkyle en C₁ à C₂₀, qui peut être substitué par un ou plusieurs substituants du groupe formé par les radicaux phényle, alkyle en C₁ à C₆, OH, halogéno ou sulfoxy.

5. Procédé selon la revendication 1, **caractérisé en ce que**, lors de la réaction énantiosélective dans un système aqueux, on utilise une solution aqueuse contenant l'hydroxynitrile lyase correspondante ou un tampon d'acétate, un tampon de borate, une solution de tampon de phtalate, de tampon de citrate, de tampon de phosphate ou un mélange de ces solutions tampons.

6. Procédé selon la revendication 5, **caractérisé en ce que** l'on ajuste dans la solution aqueuse un pH de 2 à 8.

7. Procédé selon la revendication 1, **caractérisé en ce que** l'on met en oeuvre en tant qu'agent de dilution organique des hydrocarbures aliphatiques ou aromatiques non ou peu miscibles à l'eau, qui sont éventuellement halogénés, des alcools, des éthers ou des esters, ou leurs mélanges.

8. Procédé selon la revendication 1, **caractérisé en ce que** la réaction a toutefois lieu aussi dans un système biphasique ou en émulsion.

9. Procédé selon la revendication 1, **caractérisé en ce que** l'on met en oeuvre en tant qu'hydroxynitrile lyases des (R)- et (S)-hydroxynitrile lyases natives ou recombinantes, qui sont présentes telles quelles ou immobilisées.

10. Procédé selon la revendication 9, **caractérisé en ce que** l'on met en oeuvre en tant qu'hydroxynitrile lyases des (S)-hydroxynitrile lyases natives de manioc et d'Hevea brasiliensis, des (S)-hydroxynitrile lyases recombinantes de microorganismes génétiquement modifiés du groupe formé par Pichia pastoris, E. coli ou Saccharomyces cerevisiae, des (R)-hydroxynitrile lyases natives de Prunus amygdalus, Prunus laurocerasus ou Prunus serotina, ou des (R)-hydroxynitrile lyases recombinantes.
